(19) Europäisches Patentamt / European Patent Office / Office européen des brevets

(11) **EP 2 833 471 B1**

(12) **EUROPEAN PATENT SPECIFICATION**

(45) Date of publication and mention of the grant of the patent:
**22.02.2017 Bulletin 2017/08**

(21) Application number: **13767535.1**

(22) Date of filing: **27.03.2013**

(51) Int Cl.:
***H01G 9/20*** *(2006.01)* *C09B 23/08* *(2006.01)*
*C07D 417/14* *(2006.01)* *C07D 209/18* *(2006.01)*

(86) International application number:
**PCT/JP2013/059113**

(87) International publication number:
**WO 2013/146933 (03.10.2013 Gazette 2013/40)**

(54) **DYE-SENSITIZED SOLAR CELL AND METHOD OF MANUFACTURING SAME**

FARBSTOFFSENSIBILISIERTE SOLARZELLE UND VERFAHREN ZUR HERSTELLUNG DAVON

CELLULE SOLAIRE SENSIBILISÉE PAR UN COLORANT ET PROCÉDÉ DE FABRICATION DE CETTE DERNIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2012 JP 2012082726**

(43) Date of publication of application:
**04.02.2015 Bulletin 2015/06**

(73) Proprietor: **ADEKA CORPORATION**
**Arakawa-ku**
**Tokyo**
**116-8554 (JP)**

(72) Inventors:
- **HIROSE Sadakazu**
  **Moriyama-shi, Shiga 524-8501 (JP)**
- **OKAMOTO Toshiyuki**
  **Moriyama-shi, Shiga 524-8501 (JP)**
- **MATSUNE Miwa**
  **Moriyama-shi, Shiga 524-8501 (JP)**
- **YANO Toru**
  **Arakawa-ku, Tokyo 116-8554 (JP)**
- **OSADA Hiroyuki**
  **Arakawa-ku, Tokyo 116-8554 (JP)**
- **AOYAMA Yohei**
  **Arakawa-ku, Tokyo 116-8554 (JP)**

(74) Representative: **Forstmeyer, Dietmar et al**
**BOETERS & LIECK**
**Oberanger 32**
**80331 München (DE)**

(56) References cited:
**EP-A1- 2 037 528** **WO-A1-2010/038589**
**JP-A- 2000 268 892** **JP-A- 2011 181 286**
**US-A1- 2012 012 775**

- **SAYAMA K ET AL: "Efficient sensitization of nanocrystalline TiO2 films with cyanine and merocyanine organic dyes", SOLAR ENERGY MATERIALS AND SOLAR CELLS, ELSEVIER SCIENCE PUBLISHERS, AMSTERDAM, NL, vol. 80, no. 1, 15 October 2003 (2003-10-15), pages 47-71, XP004456743, ISSN: 0927-0248, DOI: 10.1016/S0927-0248(03)00113-2**
- **GUO M ET AL: "Photoelectrochemical studies of nanocrystalline TiO2 co-sensitized by novel cyanine dyes", SOLAR ENERGY MATERIALS AND SOLAR CELLS, ELSEVIER SCIENCE PUBLISHERS, AMSTERDAM, NL, vol. 88, no. 1, 15 June 2005 (2005-06-15), pages 23-35, XP027815110, ISSN: 0927-0248 [retrieved on 2005-06-15]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 833 471 B1

## Description

### TECHNICAL FIELD

This invention relates to a dye-sensitized solar cell and a method for making the same that allow for greatly broadening the light absorption wavelength range without using a metal complex dye thereby achieving excellent photovoltaic characteristics.

### BACKGROUND ART

Dye-sensitized solar cells make use of a porous film of a metal oxide semiconductor, which is an available material, and are therefore expected to be in practical use as inexpensive devices without requiring an expensive material or processing compared with silicon-based solar cells.

The fundamental principle of dye-sensitized solar cells is as follows as discussed in patent document 1. Upon light falling on a dye-sensitized solar cell, the sensitizing dye adsorbed on the surface of a metal oxide semiconductor porous layer absorbs light to excite electrons of its molecules. The electrons are injected into the semiconductor. Thus, electrons generate in the photoelectrode, which move to the cathode through an external circuit. The electrons having moved to the cathode return to the photoelectrode through an electrolyte layer. This process is repeated to generate electrical energy to achieve high photovoltaic efficiency.

The photovoltaic efficiency of a dye-sensitized solar cell largely depends on the light absorption characteristics of a sensitizing dye. The absorption wavelength range of a sensitizing dye is related to the chemical structure of the dye. At present we have not developed a sensitizing dye exhibiting high absorption efficiency at wavelengths from the entire visible to near infrared region.

To address this problem, patent documents 2, 3, and 4 disclose approaches to improve photovoltaic efficiency of dye-sensitized solar cells by using a metal oxide semiconductor porous film having adsorbed (supported) thereon a plurality of sensitizing dyes having different absorption characteristics. Specifically, patent document 2 proposes depositing in layers at least two sensitizing dyes having different redox potentials on a metal oxide semiconductor nanoporous film in ascending order of redox potential thereby to improve photovoltaic efficiency. Patent document 3 describes a method comprising depositing at least two sensitizing dyes on a metal oxide semiconductor nanoporous film by chemical adsorption thereby to achieve high performance characteristics. Patent document 4 discloses a method comprising causing two kinds of dyes be adsorbed onto different sites of the surface of a metal oxide semiconductor nanoporous film thereby to improve the characteristics.

All the methods described above use a combination of a metal complex dye and an organic dye to be adsorbed. However, because an organic dye exhibits high adsorbability on a metal oxide semiconductor porous film, it has been difficult to cause the metal complex dye and the organic dye to be adsorbed in a well controlled manner in an attempt of broadening the absorption wavelength range thereby improving photovoltaic characteristics. Furthermore, the metal complex dye is often a complex of a rare metal, such as ruthenium, which is not only costly but may entail the resource shortage problem. In addition, when a metal oxide semiconductor is zinc oxide, a ruthenium complex dye is liable to leach from the zinc oxide film. It has therefore been sought for to develop a dye-sensitized solar cell exhibiting a broadened absorption wavelength range without the aid of a metal complex dye.

### CITATION LIST

### PATENT DOCUMENT

Patent document 1: JP 2664194
Patent document 2: JP 3505414
Patent document 3: JP 4574897
Patent document 4: JP 2009-032547A

Sayama K et al. (SOLAR ENERGY MATERIALS AND SOLAR CELLS, 80(1), pages 47-71 (2003)) report that a dye-sensitized solar cell comprising nanocrystalline $TiO_2$ films with three different dyes - yellow and red cyanine dyes, and a blue squarylium cyanine dye - adsorbed thereon has an excellent solar light-to-power conversion efficiency.

Guo M et al. (SOLAR ENERGY MATERIALS AND SOLAR CELLS, 88 (1), pages 23-35 (2005)) report about photoelectrochemical studies of nanocrystalline $TiO_2$ co-sensitized by novel cyanine dyes.

EP 2 037 528 A1 discloses a dye-sensitized solar cell comprising a combination of at least 2 different photoelectric conversion elements for a specific wavelength region, each comprising a dye or a salt thereof having a maximum absorption wavelength in a specific wavelength region supported on a thin film of oxide semiconductor fine particles provided on a substrate.

US 2012/012775 A1 discloses an electrolyte additive comprising certain benzimidazole derivatives, which electrolyte additive can increase a short circuit current density and/or solar energy-to-electricity conversion efficiency of a dye-sensitized solar cell.

SUMMARY OF THE INVENTION

PROBLEM TO BE SOLVED BY THE INVENTION

The invention provide a dye-sensitized solar cell and a method for making the same that allow for greatly broadening the light absorption wavelength range without using a metal complex dye thereby achieving excellent photovoltaic characteristics. Means for Solving the Problem

The present invention relates to a dye-sensitized solar cell including a photoelectrode having a substrate, a transparent conductive layer on the substrate, and a metal oxide porous layer composed of metal oxide particles on the transparent conductive layer, the metal oxide porous layer having sensitizing dyes adsorbed thereon. The sensitizing dyes includes an organic cyanine dye and a dye containing an indoline structure (hereinafter also referred to as an indoline dye). The organic cyanine dye and the indoline dye are adsorbed on the metal oxide porous layer in a plurality of levels. The organic cyanine dye is present in a higher concentration than the indoline dye on the surface of the metal oxide particles.

As a result of intensive investigations, the present inventors have found that a dye-sensitized solar cell having a greatly broadened absorption wavelength range and exhibiting high photovoltaic characteristics can be obtained without using a metal complex dye by causing an organic cyanine dye and an indoline dye to be adsorbed on the metal oxide porous film in a plurality of levels. The present invention has been completed based on this finding.

Fig. 1 schematically illustrates an example of the dye-sensitized solar cell of the invention.

The dye-sensitized solar cell of the invention includes a photoelectrode having a transparent substrate 1, a transparent electrode 2, and a metal oxide porous layer 5 in that order. The dye-sensitized solar cell of the invention also includes a cathode 12. The photoelectrode and the cathode 12 are superposed on each other via a seal 11 disposed along the peripheral portion of the cell. An electrolyte solution 10 is retained inside the dye-sensitized solar cell. The metal oxide porous layer 5 is composed of dye-supporting metal oxide particles 6, that is, the metal oxide porous layer 5 has a sensitizing dye supported in the pores thereof. A schematic enlarged view of the dye-supporting metal oxide particle 6 is shown in Fig. 2. In the invention, an organic cyanine dye (a first dye) 8 and an indoline dye (a second dye) 9 are adsorbed (supported) on the metal oxide particle 7 in a plurality of levels. The organic cyanine dye 8 is present closer to the metal oxide particle 7, while the indoline dye is present farther from the metal oxide particle 7. In other words, the organic cyanine dye 8 is present on the surface of the metal oxide particle 7 in a higher concentration than the indoline dye 9. In the following description, the sensitizing dye that is first adsorbed is called a first dye, and following dyes will be designated second, third, and nth dyes. As used herein, the term "in a plurality of levels" refers to two or more levels and preferably two or three levels.

The substrate that can be used in the dye-sensitized solar cell of the invention is not particularly limited, and any material that does not block incident light and has adequate strength may be used, including a glass or transparent resin film or sheet.

The transparent resin is not particularly limited and includes those having heat resistance, such as polyethylene terephthalate, polyethylene naphthalate, polysulfones, polycarbonates, polyether sulfones, polyallylates, and cyclic polyolefins.

The substrate preferably has a thickness of 20 μm to 1 mm. With the substrate thickness being within the preferred range, moderate handling properties and flexibility will be provided.

The transparent electrode may be made of a transparent oxide semiconductor, such as ITO, $SnO_2$, ZnO, GZO, AZO, and FTO. ITO is preferred for its small resistivity (providing good stability) and high transparency. The transparent electrode is formed by dry deposition techniques, such as sputtering, CVD, vapor deposition, and ion plating, or wet deposition techniques, such as a coating method using a dispersion of the oxide semiconductor particles in a liquid medium.

A metal wire mesh electrode formed by coating or printing with a dispersion of metal particles in a liquid medium may be used as a transparent electrode. Light passes through the openings of the mesh, and the metal wire functions as an electrode. A metal wire mesh electrode is very easy to make with no need of film formation equipment such as a vacuum chamber as required in, e.g., sputtering. In using a metal wire mesh electrode, it is advisable to form an anti-corrosive protective layer on the surface of the metal wire or to use an anti-corrosive metal, such as titanium or stainless steel.

A hardcoat layer may be provided between the transparent resin film and the transparent electrode to enhance adhesion of the transparent electrode or to provide protection from getting scratched.

The dye-sensitized solar cell of the invention includes a photoelectrode having the substrate, the transparent conductive layer on the substrate, and the metal oxide porous layer composed of the metal oxide particles on the transparent conductive layer.

The photoelectrode has the metal oxide porous layer. The metal oxide porous layer, being composed of metal oxide particles, is a mesoporous semiconductor film having a network of nano-sized pores.

Materials of the metal oxide particles include zinc oxide and titanium oxide. In using zinc oxide, metal oxide porous layer may be formed by coating with zinc oxide particles or electrodeposition of zinc oxide.

Methods of coating with zinc oxide particles include a process in which zinc oxide particles are dispersed in a solvent and a binder to prepare paste, which is applied by spin coating, bar coating, or printing, followed by removing the solvent. The electrodeposition method is a process in which a predetermined voltage is applied to a transparent electrode substrate in an aqueous zinc chloride solution bubbled with oxygen to plate the electrode substrate with zinc oxide. In carrying out the electrodeposition, a template material may be used to control the porosity of the zinc oxide layer.

In the case where zinc oxide is used to form the metal oxide porous layer, adhesion between zinc oxide particles is improved by treating the resulting film with warm water.

The thickness of the metal oxide porous layer is preferably 2 to 20 μm. With a thickness smaller than 2 μm, the amount of the adsorbed dye may be so small that the resulting dye-sensitized solar cell can have reduced photovoltaic characteristics. With a thickness larger than 20 μm, because the metal oxide porous layer has a limited electron diffusion length, there may be a portion that makes no contribution to photoelectric conversion, or an electrolyte solution may have difficulty in penetrating into the metal oxide porous layer, resulting in reduction of photovoltaic characteristics.

The metal oxide porous layer preferably has a porosity of 50 to 95%, more preferably 60 to 90%. When the porosity is less than 50%, it is difficult for an electrolyte solution to sufficiently penetrate into the metal oxide porous layer, resulting in reduction of photovoltaic characteristics. When the porosity exceeds 95%, the metal oxide porous layer is liable to break by an outer force due to the reduced strength. The porosity is defined by the following formula:

$$\text{Porosity} = (1 - \text{weight of metal oxide porous layer}/(\text{volume of metal oxide porous layer} \times \text{specific gravity})) \times 100\ (\%)$$

The metal oxide porous layer has a sensitizing dye adsorbed thereon and is therefore workable as a photoelectrode of a dye-sensitized solar cell in which an electromotive force is generated on irradiation with light.

The sensitizing dye comprises at least two kinds of organic dyes having different absorption wavelengths, one being an organic cyanine dye and the another one being an indoline dye (a dye having an indoline structure). These organic dyes are supported in the metal oxide porous layer in a plurality of levels to form a sensitizing dye layer. This structure allows for additively broadening the light absorption wavelength range without inviting mutual interference on their own absorption characteristics. As a result, power is generated over a broad wavelength range to provide excellent photovoltaic characteristics.

As used herein, the expression "adsorbed (or supported) in a plurality of levels" is intended to mean that sensitizing dyes comprising the organic cyanine dye and the indoline dye are adsorbed on the metal oxide porous layer through two or more steps so that the organic cyanine dye and the indoline dye are supported in layers on the metal oxide particles.

While the organic cyanine dye and the indoline dye are adsorbed on the metal oxide porous layer in a plurality of levels, it is particularly preferred that the organic cyanine dye be supported close to the metal oxide particles while the indoline dye be adsorbed with its dye skeleton being linked to the metal oxide particles via an alkylene chain. In that mode of being adsorbed, these sensitizing dyes are mutually complementary in broadening the absorption wavelength range to produce supersensitization effect on absorption wavelength broadening which is more than an additive effect. The supersensitization effect can be confirmed by, for example, the peak of incident photon-ro-current efficiency (hereinafter "IPCE") at near 700 nm that is greater than the sum of the individual contributions of the sensitizing dyes.

It is preferred for the sensitizing dye comprising the organic cyanine dye and the indoline dye to have an absorption in the entire visible region (400 to 800 nm). In terms of ease of preparation, it is preferred that at least one of the sensitizing dyes show an absorption peak at around 500 nm and that at least another one of the sensitizing dyes show an absorption peak at around 700 nm. When the absorption peak of the sensitizing dye is at a wavelength shorter or longer than the recited wavelengths, the dye-sensitized solar cell can fail to make sufficient use of visible light, the most part of sunlight.

Of the sensitizing dyes including the organic cyanine dye and the indoline dye, the one having an absorption peak at a shorter wavelength and the one having an absorption peak at a longer wavelength preferably have a difference of peak wavelength of 100 to 300 nm. When the difference is out of the range recited, the absorption wavelength range

broadening effect of the invention would be lessened.

As stated, the photoelectrode of the dye-sensitized solar cell of the invention uses organic dyes, namely, the organic cyanine dyes and the indoline dyes. Since, unlike metal complex dyes, the organic dyes do not contain a rare metal, there is no restriction on resources, pleochroism may be obtained, and an inexpensive and well-designed solar cell is produced.

The sensitizing dye comprises an organic cyanine dye. The organic cyanine dye has a large molar extinction coefficient and is therefore capable of sufficiently absorbing incident light even at a small amount and providing a dye-sensitized solar cell with a high power generation efficiency.

The organic cyanine dye is preferably selected from those having an indolenine structure at both ends of a pentamethine chain and also having a cyano group or a chloro group. Organic cyanine dyes having a pentamethine chain have an absorption in a long wavelength region of 700 to 800 nm and are used to advantage.

The organic cyanine dye preferably has at least one group selected from carboxyl, sulfo, sulfino, sulfeno, phosphono, and phosphinico. Furthermore, the organic cyanine dye preferably has a structure represented by general formula (1):

[Chemical Formula 1]

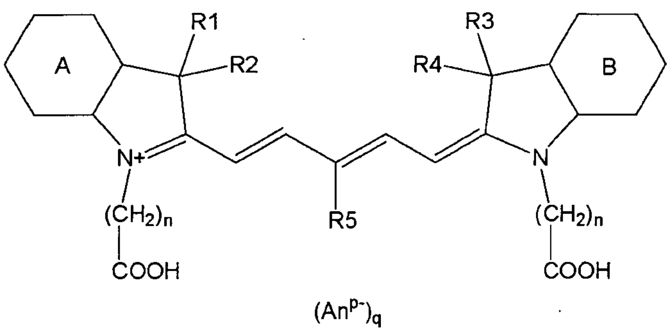

(1)

wherein ring A and ring B each independently represent an optionally substituted benzene or naphthalene ring; R1, R2, R3, and R4 each independently represent an alkyl group having 1 to 10 carbon atoms or an optionally substituted benzyl group; R5 represents a cyano group, a fluorine atom, a chlorine atom, a bromine atom, or an iodine atom; n's each independently represent an integer of 1 to 3; p represents an integer of 1 or 2; q represents an integer of 0 to 2.

In general formula (1), examples of the substituent of the rings A and B include hydroxyl, carboxyl, nitro, cyano, halogen (e.g., F, Cl, or Br), C1-C4 straight-chain or branched alkyl (e.g., methyl, ethyl, propyl, isopropyl, butyl, sec-butyl, tert-butyl), C1-C4 halogenated alkyl (e.g., $CF_3$ or $CCl_3$), C1-C4 alkoxy (e.g., methoxy, ethoxy, propoxy, isopropoxy, butoxy, sec-butoxy, or tert-butoxy), and C1-C4 halogenated alkoxy Examples of the substituents on R1, R2, R3, and R4 include those enumerated above for the rings A and B.

Of the compounds represented by general formula (1) those in which at least one of R1, R2, R3, and R4 is a benzyl group are preferred in terms of power generation characteristics. More preferred are those in which at least one of R1 and R2 and at least one of R3 and R4 are a benzyl group. Even more preferred are those in which all of R1, R2, R3, and R4 are a benzyl group.

In general formula (1), $An^{p-}$ represents a p-valent anion.

Examples of the anion represented by $An^{p-}$ include, but are not limited to, halide ions, such as fluoride ($F^-$), chloride ($Cl^-$), bromide ($Br^-$), and iodide ($I^-$); inorganic anions, such as hexafluorophosphate ($PF_6^-$), hexafluoroantimonate ($SbF_6^-$), perchlorate($ClO_4^-$), tetrafluoroborate ($BF_4^-$), chlorate, and thiocyanate; organic sulfonate anions, such as benzenesulfonate, toluenesulfonate, trifluoromethanesulfonate, diphenylamine-4-sulfonate, 2-amino-4-methyl-5-chlorobenzenesulfonate, 2-amino-5-nitrobenzenesulfonate, N-alkyldiphenylamine-4-sulfonate, and N-aryldiphenylamine-4-sulfonate; organic phosphate anions, such as octylphosphate, dodecylphosphate, octadecylphosphate, phenylphosphate, nonylphenylphosphate, and .2,2'-methylenebis(4,6-di-t-butylphenyl)phosphonate; and others, such as bistrifluoromethylsulfonylimide, bisperfluorobutanesulfonylimide, perfluoro-4-ethylcyclohexanesulfonate, tetrakis(pentafluorophenyl)borate, and tris(fluoroalkylsulfonyl)carboanions.

Examples of the anion $An^{p-}$ in general formula (1) in which p = 2, i.e., a divalent anion $An^{2-}$ include sulfate ($SO_4^{2-}$), benzenedisulfonate, and naphthalenedisulfonate.

q is a coefficient needed to keep the charge neutrality of the compound and is an integer of 0 to 2.

The sensitizing dye further comprises the indoline dye.

The indoline dye exhibits a strong absorption over a broad wavelength range in the visible region and has a

large molar extinction coefficient and is therefore capable of sufficiently absorbing incident light even at a small amount and providing a dye-sensitized solar cell with a high power generation efficiency.

It is preferred for the indoline dye to have an absorption peak at around 500 nm and has an acidic group, at which the indoline dye is adsorbed onto the metal oxide porous layer, bonded to its skeletal structure via an alkylene chain having 4 to 22 carbon atoms.

The indoline dye is preferably a compound having at least one of carboxyl, sulfo, sulfino, sulfeno, phosphono, and phosphinico groups, more preferably a compound represented by general formula (2):

[Chemical Formula 2]

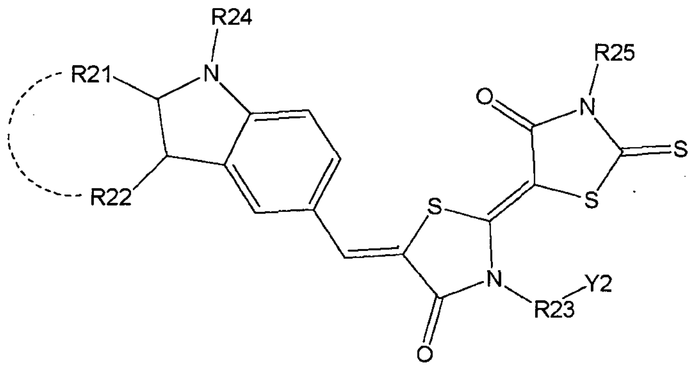

(2)

wherein R21 and R22 each represent a hydrogen atom or an alkyl group, or R21 and R22 are taken together to form a cyclopentane ring or a cyclohexane ring; R23 represents an alkylene group having 1 to 3 carbon atoms; Y2 represents at least one group selected from carboxyl, sulfo, sulfino, sulfeno, phosphono, and phosphinico; R24 represents an aliphatic hydrocarbon residue, an aromatic hydrocarbon residue, or a heterocyclic residue; R25 represents an alkyl group or an aralkyl group, provided that at least one of R24 and R25 contains at least one group selected from carboxyl, sulfo, sulfino, sulfeno, phosphono, and phosphinico bonded via an alkylene group having more than 3 carbon atoms.

In general formula (2), examples of the alkyl group represented by R21 and R22 are methyl, ethyl, n-butyl, and n-octyl. R21 and R22 may be taken together to form a cyclopentane or a cyclohexane ring.

R23 represents a C1-C3 alkylene group, preferably a C1-C2 alkylene group.

Y2 represents an acidic group having a pKa of smaller than 6. Examples of the acidic group having a pKa of smaller than 6 include carboxyl, sulfo, sulfino, sulfeno, phosphono, and phosphinico, with carboxyl being particularly preferred.

R24 is an aliphatic hydrocarbon residue, an aromatic hydrocarbon residue, or a heterocyclic residue.

Examples of the aliphatic hydrocarbon residue include alkyl groups, such as methyl, ethyl, propyl, and octyl; alkenyl groups, such as allyl and butenyl; alkynyl groups, such as propargyl; and aralkyl groups, such as benzyl and phenethyl. Examples of the aromatic hydrocarbon residue include phenyl, tolyl, and naphthyl. Examples of the heterocyclic residue are indolyl, pyridyl, furyl, and thienyl. Preferred of these groups are aromatic hydrocarbon residues.

The aliphatic hydrocarbon, aromatic hydrocarbon, and heterocyclic residues may further be substituted by various substituents. Examples of preferred substituents include the above described aliphatic hydrocarbon, aromatic hydrocarbon, and heterocyclic residues and, in addition, amino, vinyl, alkoxy, aryloxy, alkylthio, arylthio, hydroxyl, halogen, and an acidic group having a pKa of smaller than 6.

Of the aromatic hydrocarbon residues enumerated above for R24, preferred residues include, but are not limited to, AS-1 through AS-25 shown below.

[Chemical Formula 3]

AS-1

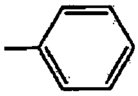

AS-2

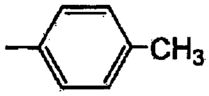

AS-3

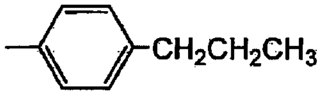

AS-4

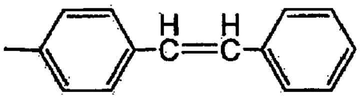

AS-5

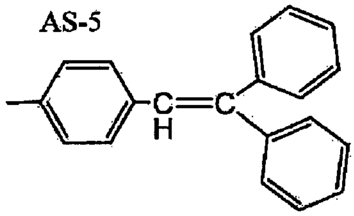

AS-6

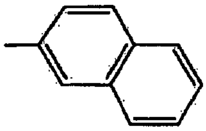

AS-7

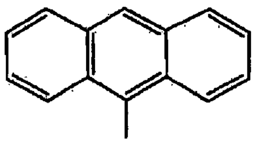

AS-8

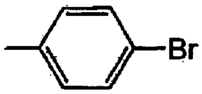

AS-9

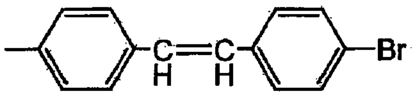

AS-10

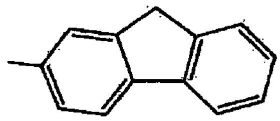

AS-11

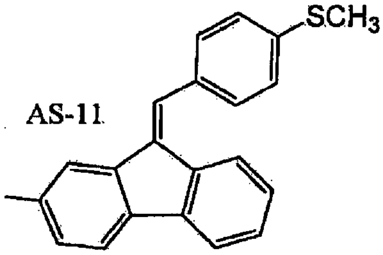

AS-12

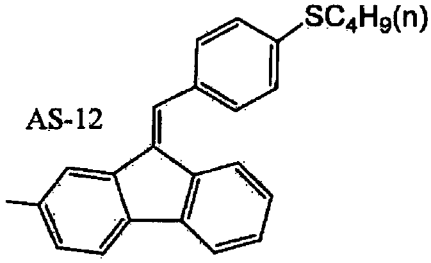

[Chemical Formula 4]

AS-13

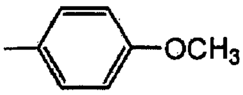

AS-14

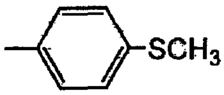

AS-15

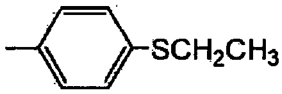

AS-16

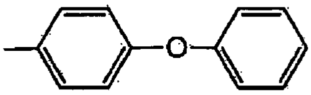

AS-17

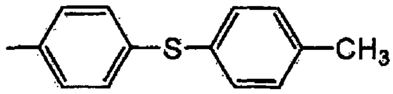

AS-18

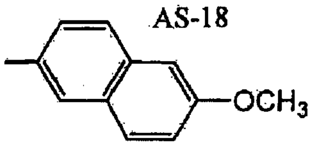

AS-19

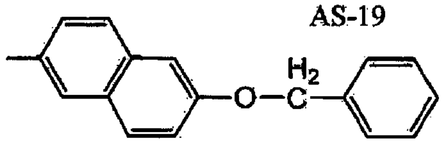

AS-20

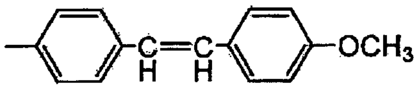

AS-21

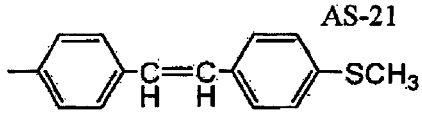

AS-22

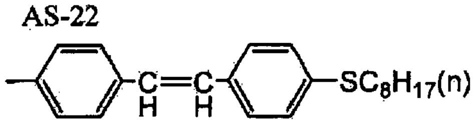

AS-23

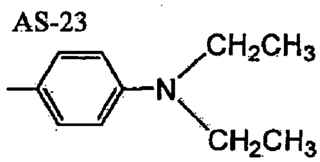

AS-24

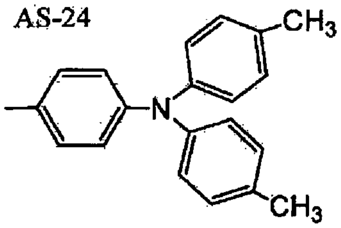

AS-25

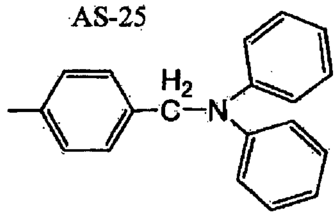

Of the aromatic hydrocarbon residues AS-1 to AS-25, particularly preferred are AS-5, AS-10 to AS-15, and AS-20 to AS-22 in terms of photovoltaic efficiency.

R25 represents an alkyl group or an aralkyl group.

Examples of the alkyl group include methyl, ethyl, propyl, octyl, pentyl, hexyl, octyl, decyl, and dodecyl. They may be either linear or branched. Preferred of them are linear C5-C14 alkyl groups. Examples of the aralkyl group include benzyl, phenethyl, and 1-naphthylmethyl.

At least one of R24 and R25 contains an acidic group having a pKa of smaller than 6, the acidic group being bonded via an alkylene group having more than 3 carbon atoms. Examples of the acidic group having a pKa of less than 6 that is bonded via an alkylene group having more than 3 carbon atoms include AC-41 to AC-60 shown below. A preferred upper limit of the number of carbon atoms of the alkylene group is 22. *Inter alia*, an acidic group having a pKa of less than 6 bonded via a linear C4-C14 alkylene group is preferred. Examples of the acidic group having a pKa of less than 6 are the same as those listed above.

[Chemical Formula 5]

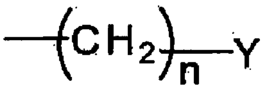

| | | | |
|---|---|---|---|
| AC-41 | n=4 | AC-51 | n=14 |
| AC-42 | n=5 | AC-52 | n=15 |
| AC-43 | n=6 | AC-53 | n=16 |
| AC-44 | n=7 | AC-54 | n=17 |
| AC-45 | n=8 | AC-55 | n=18 |
| AC-46 | n=9 | AC-56 | n=19 |
| AC-47 | n=10 | AC-57 | n=20 |
| AC-48 | n=11 | AC-58 | n=21 |
| AC-49 | n=12 | AC-59 | n=22 |
| AC-50 | n=13 | AC-60 | n=23 |

The sensitizing dye may further comprise an organic dye other than the organic cyanine dye and the indoline dye. The organic dyes other than the organic cyanine dye and the indoline dye are not particularly limited as long as they are capable of absorbing light energy to generate electrons and swiftly injecting the electrons to the metal oxide porous layer. Those having a functional group are preferred to be firmly adsorbed onto the metal oxide porous layer. Examples of such a functional group include carboxyl, carboxylic acid anhydride, alkoxy, hydroxyl, hydroxyalkyl, sulfo, ester, mercapto, and sulfonyl.

Examples of the other organic dyes include xanthene dyes, such as Eosin Y, Fluorescein, Erythrosine B, Phloxine B, Rose Bengal, Fluorexon, Merbromin, Dibromofluorescein, and pyrogallol red; coumarin dyes, such as coumarin 343; triphenylmethane dyes, such as Bromophenol Blue, Bromothymol Blue, and phenolphthalein; indigo dyes, oxonol dyes, porphyrine dyes, phthalocyanine dyes, azo dyes, quinone dyes, quinoneimine dyes, squarylium dyes, perylene tetracarboxylic acid derivatives; and natural colorants, such as anthocyanins, gardenia pigments, turmeric pigment, safflower pigment, carotenoids, cochineal pigment, and paprika pigment.

The photoelectrode, electrolyte layer, and cathode are stacked in that order to make a dye-sensitized solar cell. More specifically, a solution containing an electrolyte is dropped or applied on the photoelectrode to form an electrolyte layer, and a cathode is then placed thereon, or the photoelectrode and a cathode having an inlet for injecting an electrolyte solution are stacked, and an electrolyte solution is poured through the inlet.

The electrolyte layer may be either of an electrolyte solution or a semisolid electrolyte prepared using a gelling agent. Any substance capable of transporting electrons, holes, ions, and the like may be used as the electrolyte layer, including solid hold-transport materials (p-type semiconductors), such as CuI, CuSCN, NiO, $Cu_2O$, and KI; and solutions of reduction/oxidation couples (redox electrolytes), such as iodine/iodide and bromine/bromide, in an organic solvent. A solution of a redox electrolyte in an organic solvent is preferred; for it easily penetrates into the inside of the metal oxide porous layer and hardly desorbs the adsorbed dyes from the metal oxide porous layer.

Examples of the gelling agent include dibenzylidene D-sorbitol, cholesterol derivatives, amino acid derivatives, trans-(1R,2R)-1,2-cyclohexanediamine alkylamide derivatives, alkylurea derivatives, N-octyl-D-gluconamide benzoate, double-headed amino acid derivatives, quaternary ammonium derivatives, layered clay minerals, such as smectite clay minerals and swellable mica (see JP 4692694, para. [0044]-[0065]), and photopolymerizable monomers, such as acrylic acid monomers.

Examples of the organic solvent include nitriles, such as acetonitrile, methoxypropionitrile, butyronitrile, methoxyacetonitrile, and valeronitrile; hydrocarbons, such as propylene carbonate, diethyl carbonate, γ-butyrolactane, N-methylpyrrolidone, tetrahydrofuran, dimethyl carbonate, ethylmethyl carbonate, ethylene carbonate, and 1,4-dioxane; alcohols, such as butanol, pentanol, and polyethylene glycol; N,N-dimethylformamide; quinoline; and ionic liquids, such as imidazolium salts, pyrrolidinium salts, piperidinium salts, and pyridinium salts. These organic solvents may be used either individually or in combination of two or more thereof.

As the redox electrolyte, known electrolytes may be used, such as redox electrolytes having an oxidation-reduction couple. Examples of the redox electrolyte include $I^-/I_3^-$ couples, $Br^-/Br_3^-$ couples, quinone/hydroquinone couples, Co complexes, and nitroxy radical compounds. Specifically included are combinations of a halogen and a halide,

such as iodine/iodide couples and bromine/bromide couples. Examples of the halide include cesium halides, quaternary alkylammonium halides, imidazolium halides, thiazolium halides, oxazolium halides, quinolinium halides, and pyridinium halides. More specifically, examples of the iodides include cesium iodide; quaternary alkylammonium iodides, such as tetraethylammonium iodide, tetrapropylammonium iodide, tetrabutylammonium iodide, tetrapentylammonium iodide, tetrahexylammonium iodide, tetraheptylammonium iodide, and trimethylphenylammonium iodide; imidazolium iodides, such as 3-methylimidazolium iodide and 1-propyl-2,3-dimethylimidazolium iodide; thiazolium iodides, such as 3-ethyl-2-methyl-2-thiazolium iodide, 3-ethyl-5-(2-hydroxyethyl)-4-methylthiazolium iodide, and 3-ethyl-2-methylbenzothiazolium iodide; oxazolium iodides, such as 3-ethyl-2-methylbenzoxazolium iodide; quinolinium iodides, such as 1-ethyl-2-methylquinolinium iodide; and pyridinium iodides. Examples of the bromides include quaternary alkylammonium bromides. Preferred of the halogen/halide couples are couples of iodine and at least one of the above described iodides.

The redox electrolyte may be a combination of an ionic liquid and a halogen. In this case, the redox electrolyte may further contain the above described halide. Examples of the ionic liquid include those usable in electric batteries and solar cells, such as those disclosed in Inorg. Chem., 1996, 35, pp. 1168-1178, Electrochemistry, 2002, 2, pp. 130-136, JP 9-507334A, and JP 8-259543A. Preferred of them are salts whose melting point is below room temperature (25°C) or salts the melting point of which is higher than room temperature but which are liquefied at room temperature on dissolving with other fused salt. Specific examples of the ionic liquids are represented by anions and cations described below.

Examples of cations of ionic liquids are ammonium, imidazolium, oxazolium, thiazolium, oxadiazolium, triazolium, pyrrolidinium, pyridinium, piperidinium, pyrazolium, pyrimidinium, pyradinium, triazinium, phosphonium, sulfonium, carbazolium, indolium, and derivatives thereof. They may be used either individually or as a mixture of two or more thereof. Specific examples include 1-methyl-3 -propylimidazolium, 1-butyl-3 -methylimidazolium, 1,2-dimethyl-3-propylimidazolium, and 1-ethyl-3-methylimidazolium.

Examples of anions of ionic liquids include metal chloride ions, e.g., $AlCl_4^-$ and $Al_2Cl_7^-$; fluorine-containing anions, such as $PF_6^-$, $BF_4^-$, $CF_3SO_3^-$, $N(CF_3SO_2)_2^-$, $F(HF)_n^-$, and $CF_3COO^-$; fluorine-free anions, such as $NO_3^-$, $CH_3COO^-$, $C_6H_{11}COO^-$, $CH_3OSO_3^-$, $CH_3OSO_2^-$, $CH_3SO_3^-$, $CH_3SO_2^-$, $(CH_3O)_2PO_2^-$, $N(CN)_2^-$, and $SCN^-$; and other halide ions, such as iodide ions and bromide ions. These anions may be used either individually or as a mixture of two or more thereof. Preferred of these anions of ionic liquids are iodide ions.

For the purpose of improving power generation efficiency, durability, and the like of the photoelectric device, the electrolyte layer may contain acyclic saccharides (see JP 2005-093313A), pyridine compounds (see JP 2003-331936A), urea derivatives (see JP 2003-168493A), and so on.

The cathode is not particularly limited. For example, a stack of the same substrate and the same transparent electrode as used in the photoelectrode and a catalyst layer in that order may be used. The electrode of the cathode does not always need to have transparency and may be made of an anti-corrosive metal, such as titanium or tungsten, a carbon material, such as graphite, or conductive polymers, such as PEDOT/PSS. The catalyst layer may be made of platinum, carbon, or a conductive polymer, such as polythiophene or polyaniline.

The photoelectrode and the cathode are put together to make a cell, and a seal is provided along the cell periphery so that an electrolyte may be retained inside. While the seal may be made of various adhesives or pressure-sensitive adhesives, the sealing material should be non-reactive with the electrolyte solution and inert to the solvent of the electrolyte solution. silicone-based or fluorine-containing adhesives or pressure-sensitive adhesives having good adhesion to the film substrate are suitably used. Fusion bonding using an ionomer resin film is also suitable.

The method for making the dye-sensitized solar cell of the invention is not particularly limited as long as it includes the step of causing the sensitizing dyes comprising the organic cyanine dye and the indoline dye to be adsorbed onto the metal oxide porous layer in a plurality of levels. For example, the dye-sensitized solar cell of the invention is obtained by a method including the steps of providing a substrate having a transparent conductive layer thereon, forming a metal oxide porous layer of metal oxide particles on the transparent conductive layer, causing the organic cyanine dye to be adsorbed to the metal oxide porous layer, and causing the indoline dye to be adsorbed to the metal oxide porous layer having the organic cyanine dye adsorbed thereon. This method for making the dye-sensitized solar cell constitutes another aspect of the present invention.

The method for making a dye-sensitized solar cell according to the invention includes the step of forming a metal oxide porous layer on the transparent conductive layer formed on the substrate. Specifically, this step may be carried out by dispersing zinc oxide particles in a mixture of a solvent and a binder to prepare paste, printing the paste on the transparent conductive layer, and removing the solvent by drying. A three-electrode method may also be employed, in which the substrate having a transparent electrode is immersed in an electrolytic solution containing a zinc salt and a template compound, setting the transparent electrode as a working electrode and zinc as a counter electrode in the electrolytic solution, and applying a constant negative voltage to the reference electrode while bubbling with oxygen.

Examples of the zinc salt include, but are not limited to, $ZnCl_2$, $ZnBr_2$, and $ZnI_2$. The lower and the upper limit of the zinc salt concentration in the electrolytic solution are preferably 1 mM/L and 50 mM/L, respectively. At concentrations lower than 1 mM/L, the electrolytic solution can fail to form an adequate thin film for a dense zinc oxide layer and for a

porous zinc oxide layer. Where the concentration is higher than 50 mM/L, oxygen supply to zinc can be insufficient, resulting in precipitation of metallic zinc.

The template compound, which is added to the electrolytic solution containing the zinc salt, is a compound adsorbed to the internal surface of a metal oxide porous layer formed by electrodeposition and released afterward by a prescribed desorption means. While the template compound is not particularly limited as long as it has the above described properties and is soluble in the electrolytic solution such as an aqueous zinc salt solution, an organic compound having a $\pi$ electron, such as an aromatic compound having electrochemical reducing properties is suitable. In particular, a xanthene dye, which is an organic dye, is preferred, such as Eosin Y, Erythrosine Y, Phloxine B, Rose Bengal, or Rhodamine B.

The electrolytic solution containing the zinc salt and the template compound may further contain appropriate additives for prevention of agglomeration or for other purposes, such as a surfactant.

After the electrodeposition, the template compound is desorbed to leave a metal oxide porous layer. The method for desorbing the template compound is not particularly limited, and any method appropriate to the template compound may be chosen. In using, for example, a template compound having such an anchor group as carboxyl, sulfo, or phosphate group, it may be released by washing with an alkali (e.g., sodium hydroxide or potassium hydroxide) solution.

The method of the invention preferably includes the steps of causing the organic cyanine dye to be adsorbed to the metal oxide porous layer and causing the indoline dye to be adsorbed to the metal oxide porous layer having the organic cyanine dye adsorbed thereon.

The organic cyanine dye used in the above method is preferably the compound having the structure represented by general formula (1) described *supra.*

The indoline dye used in the method is preferably a compound having a bonding group at the end of a long-chain alkyl group, particularly the compound having the structure represented by general formula (2). It is generally difficult to make an organic dye be adsorbed onto an adsorbent after a different organic dye is adsorbed to the adsorbent. In the case of the indoline dye having the structure of general formula (2), which has a bonding group at the long-chain alkyl terminal, adsorption is advantageously achieved without interfering with the characteristics of the previously adsorbed sensitizing dye (organic cyanine dye).

Adsorption of the sensitizing dye is carried out by, for example, immersing a resin film substrate having the metal oxide porous layer thereon in a solution containing the sensitizing dye, followed by drying, and repeating the immersion-drying cycles a required number of times.

The concentration of the dye solution is preferably 0.05 to 3.0 mM, more preferably 0.1 to 1.0 mM. The amount of the adsorbed sensitizing dye may be too small to exhibit sufficient characteristics at concentrations lower than 0.05 mM. The sensitizing dye may be adsorbed in the form of aggregations at concentrations higher than 3.0 mM.

The solvent used in the sensitizing dye solution may be any solvent that is capable of dissolving the dye and does not deteriorate the substrate. Examples of suitable solvents are alcohols, such as ethanol and butanol, ketones, such as acetone, ethers, such as diethyl ether, and acetonitrile. These solvents may be used either individually or as a mixture of two or more thereof. Ethanol or a mixed solvent of butanol and acetonitrile is preferred.

## EFFECT OF THE INVENTION

The invention allows for greatly broadening the absorption wavelength range without using a metal complex dye by causing an organic cyanine dye and an indoline dye to be adsorbed onto the metal oxide porous film in a plurality of levels and therefore provides a dye-sensitized solar cell exhibiting high photovoltaic characteristics.

## BRIEF DESCRIPTION OF THE DRAWINGS

Fig. 1 schematically illustrates an example of the dye-sensitized solar cell of the invention.

Fig. 2 is an enlarged schematic view illustrating an example of the dye -sensitized solar cells of the invention.

Fig.3 is a graph of IPCE of the dye-sensitized solar cells obtained in Example 1, Comparative Example 1, and Comparative Example 2.

## BEST MODE FOR CARRYING OUT THE INVENTION

The invention will now be illustrated in greater detail with reference to Examples, but it should be understood that the invention is not limited thereto.

Example 1

(1) Step of zinc oxide porous layer formation

A 200 μm thick PEN film from Teijin Du Pont Films was used as a transparent resin substrate. An ITO transparent electrode was formed on the substrate by DC sputtering under the following conditions: argon gas flow rate of 50 sccm, oxygen gas flow rate of 1.5 sccm, voltage of 370 V, current of 2A, and sputtering time of 20 minutes. The resulting ITO transparent electrode had a surface resistivity of 21 Ω/sq.

Zinc oxide particles MZ-500 from Tayca Corp. (average particle size: 25 nm) were dispersed in a mixture of terpineol as a solvent and ethyl cellulose as a binder to prepare paste. The paste was screen printed on the ITO transparent electrode, followed by solvent removal by drying. The screen printing was carried out using a pattern having 15 circular openings of 6 mm in diameter. The solvent removal was carried out at 100°C for 30 minutes. The resulting zinc oxide porous layer had a thickness of 10 μm and a porosity of 61.3%. The zinc oxide porous layer was treated with warm water by immersion in water at 60°C for 10 minutes, dried at 100°C for 30 minutes, and then subjected to UV cleaning using a low pressure mercury lamp (254 nm).

(2) Step of sensitizing dye adsorption

An organic cyanine dye of formula (3) below (blue dye from ADEKA Corp.; absorption peak: 680 nm), designated cyanine dye 1, was dissolved in ethanol to prepare a 0.2 mM first dye solution. The substrate having the zinc oxide porous layer was immersed in the first dye solution for 120 minutes to cause the first dye to be adsorbed to the zinc oxide porous layer.

Subsequently, an indoline dye of formula (4) below (red dye from Chemicrea Inc.; absorption peak: 540 nm), designated indoline dye 1, and cholic acid were dissolved in a 1:1 mixed solvent of t-butanol and acetonitrile to prepare a second dye solution containing 0.2 mM indoline dye 1 and 0.4 mM cholic acid. The substrate with the zinc oxide layer was immersed in the second dye solution for 120 minutes to cause the second dye to be adsorbed to the zinc oxide porous layer. A photoelectrode was thus prepared.

[Chemical Formula 6]

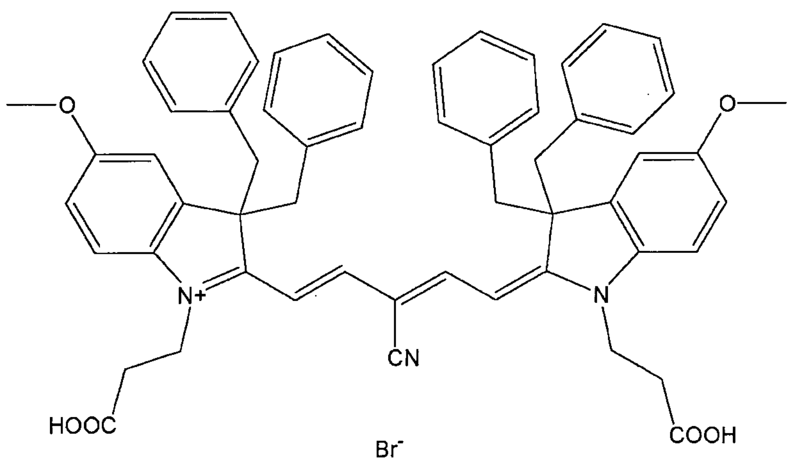

(3)

[Chemical Formula 7]

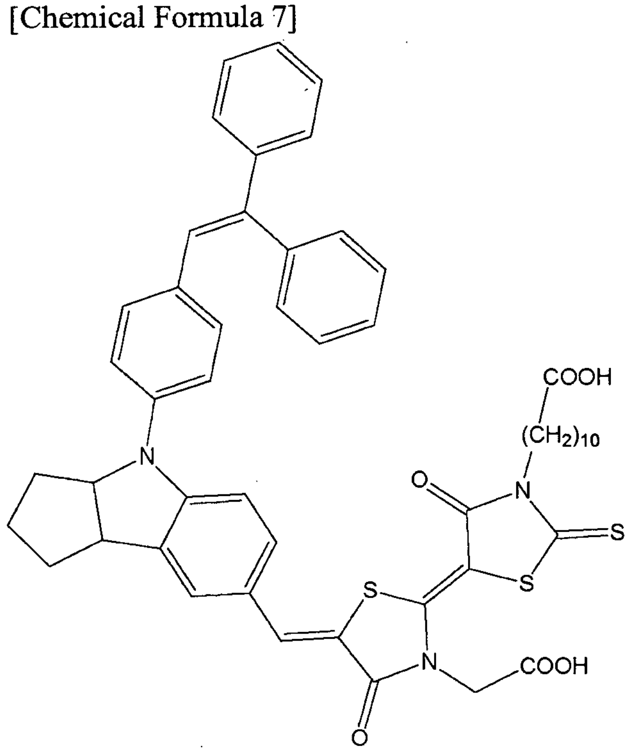

(4)

(3) Confirmation of adsorbed state of sensitizing dyes

The chromaticity (a*) of the zinc oxide porous layer having the sensitizing dyes adsorbed thereon was measured using a spectrophotometer CM-3600d from Konica Minolta, Inc. After the zinc oxide porous layer was immersed in N,N-dimethylacetamide for 30 hours, the chromaticity (a*) was measured again. A difference in chromaticity (Δa*) was calculated, which indicates the degree of desorption of the red dye. The adsorbed state of the sensitizing dye was evaluated based on this chromaticity difference. As a result, the zinc oxide porous layer obtained in the step of sensitizing dye adsorption in Example 1 showed a chromaticity difference (Δa*) of -17.64. The considerable reduction in chromaticity (a*) indicated desorption of the red dye. It is seen from this result that indoline dye 1 had been supported outside of cyanine dye 1 and that cyanine dye 1 was present on the surface of the particles in a higher concentration than indoline dye 1.

The zinc oxide porous layer having the sensitizing dyes adsorbed thereon was further analyzed by elemental analysis using energy dispersive X-ray spectroscopy (EDX) under SEM in the depth direction from the interface of the zinc oxide porous layer to examine the ratio of sulfur contained in the indoline structure of the indoline dye. As a result, a relatively large amount of sulfur was detected near the interface (outer surface) of the zinc oxide porous layer while a relatively small amount of sulfur was detected at distances from the interface (inside of the outer surface), proving that the indoline dye was present in a larger amount on the outer side of the zinc oxide porous layer and in a smaller amount inside of the interface, i.e., on the surface of the zinc oxide particles. These results also support the consideration that cyanine dye 1 was present on the surface of the zinc oxide particles in a higher concentration than indoline dye 1.

(4) Assembly of dye-sensitized solar cell

A UV-curing adhesive TB3035B from Three Bond Co., Ltd. was printed on the photoelectrode in a prescribed pattern surrounding the zinc oxide porous layer.

Separately, a cathode was made as follows. An ITO electrode film was formed on a PEN film, and a platinum catalyst layer was formed on the ITO electrode by DC sputtering under the following conditions: argon gas flow rate of 30 sccm, voltage of 560 V, current of 2.8 A, and sputtering time of 1 minute. The surface resistivity was 7 Ω/sq. The thus processed substrate was cut to a predetermined shape to make cathodes.

A predetermined amount of an electrolyte solution containing 0.1 mol/L iodine and 1.0 mol/L 1,2-dimethyl-3-propylimidazolium iodide in an imidazolium salt ionic liquid (IL120 from Dai-ichi Kogyo Seiyaku Co., Ltd.) as a solvent was dropped on the zinc oxide porous layer of the photoelectrode using a micropipette, and the cathode was stuck to the photoelectrode via the adhesive to make a 35 mm × 35 mm dye-sensitized solar cell having a power generating area of 8 mm in diameter.

Example 2

A dye-sensitized solar cell was made in the same manner as in Example 1, except for replacing indoline dye 1 used in the step of sensitizing dye adsorption with indoline dye 2 represented by formula (5) below (D131 from Chemicrea; absorption peak: 440 nm).

[Chemical Formula 8]

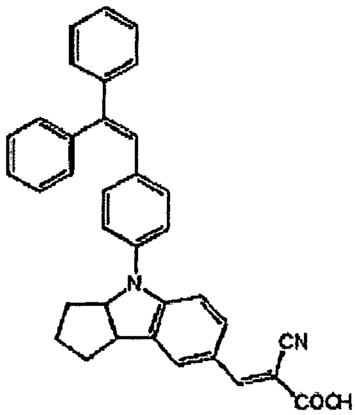

(5)

Example 3

A dye-sensitized solar cell was made in the same manner as in Example 1, except that the step of sensitizing dye adsorption was carried out as follows. Cyanine dye 1 and indoline dye 1 were dissolved in a 1:1 mixed solvent of t-butanol and acetonitrile to prepare a mixed solution containing 0.2 mM organic cyanine dye 1 and 0.2 mM indoline dye 1, and the substrate having the zinc oxide porous layer was immersed in the mixed solution for 120 minutes to cause the sensitizing dyes to be adsorbed on the zinc oxide porous layer.

Confirmation of adsorbed state of sensitizing dyes:

The adsorbed state of sensitizing dyes was examined in the same manner as in Example 1. As a result, the zinc oxide porous layer of Example 3 showed a chromaticity difference ($\Delta a^*$) of -16.95. The considerable reduction in chromaticity ($a^*$) indicated desorption of the red dye. It is seen from this result that indoline dye 1 had been supported outside of cyanine dye 1 and that cyanine dye 1 was present on the surface of the particles in a higher concentration than indoline dye 1.

The zinc oxide porous layer was further analyzed by EDX under SEM in the depth direction of the zinc oxide porous layer to examine the ratio of sulfur contained in the indoline structure of the indoline dye in the same manner as in Example 1. As a result, it was revealed that indoline dye was present in a larger amount on the outer surface of the zinc oxide porous layer and in a smaller amount inside of the outer surface of the porous layer. These results also support the consideration that cyanine dye 1 was present on the surface of the zinc oxide particles in a relatively high concentration.

Example 4

A dye-sensitized solar cell was made in the same manner as in Example 1, except for replacing cyanine dye 1 used in the step of sensitizing dye adsorption with organic cyanine dye 2 represented by formula (6) below (from ADEKA; absorption peak: 680 nm).

[Chemical Formula 9]

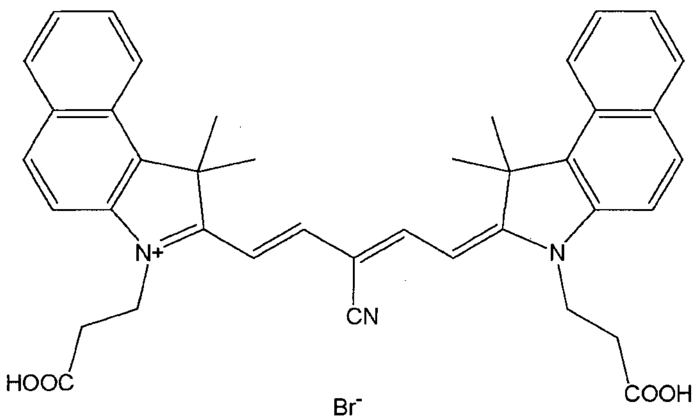

(6)

Comparative Example 1

A dye-sensitized solar cell was made in the same manner as in Example 1, except that only cyanine dye 1 was caused to be adsorbed on the zinc oxide porous layer but indoline dye 1 was not in the step of sensitizing dye adsorption.

Comparative Example 2

A dye-sensitized solar cell was made in the same manner as in Example 1, except that only indoline dye 1 was caused to be adsorbed on the zinc oxide porous layer but cyanine dye 1 was not in the step of sensitizing dye adsorption.

Comparative Example 3

A dye-sensitized solar cell was made in the same manner as in Example 1, except that the step of sensitizing dye adsorption was carried out as follows.

Indoline dye 1 (from Chemicrea) and cholic acid were dissolved in a 1:1 mixed solvent of t-butanol and acetonitrile to prepare a first dye solution containing 0.2 mM indoline dye 1 and 0.4 mM cholic acid. The substrate having the zinc oxide porous layer was immersed in the first dye solution for 120 minutes to cause the first dye to be adsorbed to the zinc oxide porous layer.

Subsequently, cyanine dye 1 (from ADEKA) was dissolved in ethanol to prepare a 0.2 mM second dye solution. The substrate with the zinc oxide layer was immersed in the second dye solution for 120 minutes to cause the second dye to be adsorbed onto the zinc oxide porous layer. A photoelectrode was thus prepared.

Confirmation of adsorbed state of sensitizing dyes:

The adsorbed state of sensitizing dyes was examined in the same manner as in Example 1. As a result, the zinc oxide porous layer of Comparative Example 3 showed a chromaticity difference ($\Delta a^*$) of 15.27, i.e., a considerable increase in chromaticity ($a^*$). This is believed to be due to desorption of the blue dye but not of the red dye. It is seen from this result that cyanine dye 1 had been supported outside of indoline dye 1 and that indoline dye 1 was present on the surface of the particles in a higher concentration than cyanine dye 1.

The zinc oxide porous layer was further analyzed by EDX under SEM in the depth direction of the zinc oxide porous layer to examine the ratio of sulfur contained in the indoline structure of the indoline dye in the same manner as in Example 1. As a result, it was confirmed that the indoline dye was present in a smaller amount in the outer surface of the zinc oxide porous layer and in a larger amount inside of the outer surface of the porous layer. These results also support the consideration that indoline dye 1 was distributed on the surface of the zinc oxide particles in a relatively high concentration.

Comparative Example 4

A dye-sensitized solar cell was made in the same manner as in Example 2, except that only indoline dye 2 was caused to be adsorbed onto the zinc oxide porous layer but cyanine dye 1 was not used in the step of sensitizing dye

adsorption.

Comparative Example 5

A dye-sensitized solar cell was made in the same manner as in Example 4, except that only cyanine dye 2 was caused to be adsorbed onto the zinc oxide porous layer but indoline dye 1 was not in the step of sensitizing dye adsorption.

Evaluation:

Evaluation was performed according to the following procedures. The results obtained are shown in Table 1 below.

(1) Photovoltaic characteristics

The dye-sensitized solar cells obtained in Examples and Comparative Examples were evaluated for photovoltaic characteristics in terms of open circuit voltage (Voc), short circuit current (Jsc), fill factor (FF), and conversion efficiency ($\eta$) using a solar simulator having a light source intensity of 1 sun (=100 mW/cm$^2$).

The dye-sensitized solar cells of Example 1 and Comparative Examples 1 and 2 were tested using a spectral sensitivity measurement system to measure IPCE. The results obtained are shown in Fig. 3. For comparison, the IPCE of the Comparative Example 1 using cyanine dye 1 alone and Comparative Example 2 using indoline dye 1 alone are also shown.

Table 1

| | Cell Characteristics | | | |
|---|---|---|---|---|
| | Jsc (mA/cm$^2$) | Voc (mV) | FF | $\eta$ (%) |
| Example 1 | 10.27 | 547 | 0.644 | 3.62 |
| Example 2 | 5.64 | 498 | 0.624 | 1.76 |
| Example 3 | 7.48 | 523 | 0.621 | 2.43 |
| Example 4 | 9.64 | 477 | 0.642 | 2.95 |
| Comparative Example 1 | 4.79 | 486 | 0.697 | 1.62 |
| Comparative Example 2 | 5.51 | 550 | 0.711 | 2.16 |
| Comparative Example 3 | 3.67 | 513 | 0.712 | 1.34 |
| Comparative Example 4 | 2.61 | 479 | 0.648 | 0.81 |
| Comparative Example 5 | 1.76 | 415 | 0.654 | 0.48 |

As is shown in Table 1, the dye-sensitized solar cells obtained in Examples 1 to 4 exhibit high photovoltaic efficiency. With respect to wavelength-dependency of IPCE, the IPCE of the dye-sensitized solar cell of Example 1 is higher than the additive level of each sensitizing dye, proving that the supersensitization effect used in the silver salt photographic technology is obtained.

In contrast, the dye-sensitized solar cells of Comparative Examples 1 to 5 have insufficient photovoltaic characteristics. The reason the dye-sensitized solar cell of Comparative Example 3 has particularly poor photovoltaic characteristics is believed to be because the indoline dye that has been adsorbed before the organic cyanine dye hinders the organic cyanine dye from being adsorbed and, as a result, the concentration of the indoline dye is higher than that of the organic cyanine dye on the surface of the metal oxide particles. On the other hand, the high photovoltaic characteristics of the dye-sensitized solar cell of Example 3 is believed to be because the organic cyanine dye is adsorbed directly onto the surface of the metal oxide particles while the indoline dye is adsorbed via the long-chain alkyl group and, as a result, the concentration of the organic cyanine dye is higher than that of the indoline dye on the surface of the metal oxide particles.

INDUSTRIAL APPLICABILITY

The invention provide a dye-sensitized solar cell and a method for making the same that allow for greatly

broadening the light absorption wavelength range without using a metal complex dye thereby achieving excellent photovoltaic characteristics.

DESIGNATION OF REFERENCE NUMERALS

1 transparent substrate
2 transparent electrode
5 metal oxide porous layer
6 dye-supporting metal oxide particle
7 metal oxide particle
8 organic cyanine dye
9 indoline dye containing indoline structure
10 electrolyte solution
11 seal
12 cathode

## Claims

1. A dye-sensitized solar cell comprising a photoelectrode comprising a substrate (1), a transparent conductive layer (2) on the substrate (1), and a metal oxide porous layer (5) containing metal oxide particles (6/7) on the transparent conductive layer (2), the metal oxide porous layer (5) having sensitizing dyes adsorbed thereon, **characterized in that** the sensitizing dyes comprising an organic cyanine dye (8) and an indoline dye containing an indoline structure (9), the organic cyanine dye (8) and the indoline dye (9) being adsorbed onto the metal oxide porous layer (5) in a plurality of levels, and
the organic cyanine dye (8) being present in a higher concentration than the indoline dye (9) on the surface of the metal oxide particles (7).

2. The dye-sensitized solar cell according to claim 1, wherein the organic cyanine dye (8) and the indoline dye (9) each have at least one group selected from carboxyl, sulfo, sulfino, sulfeno, phosphono, and phosphinico.

3. The dye-sensitized solar cell according to claim 1 or 2, wherein the organic cyanine dye (8) has a structure represented by general formula (1):

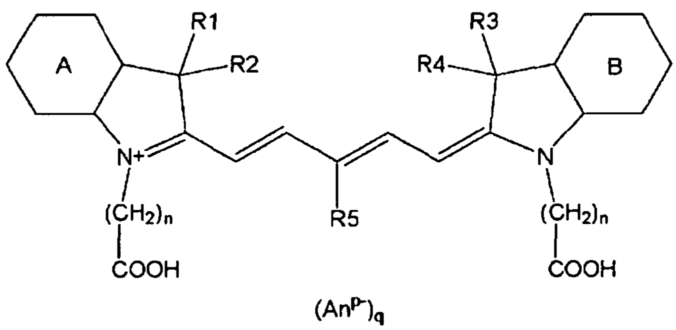

(1)

wherein ring A and ring B each independently represent an optionally substituted benzene or naphthalene ring; R1, R2, R3, and R4 each independently represent an alkyl group having 1 to 10 carbon atoms or an optionally substituted benzyl group; R5 represents a cyano group, a fluorine atom, a chlorine atom, a bromine atom, or an iodine atom; n's each independently represent an integer of 1 to 3; $An^{p-}$ represents a p-valent anion, p represents an integer of 1 or 2; q represents an integer of 0 to 2.

4. The dye-sensitized solar cell according to claim 1, 2, or 3, wherein the indoline dye (9) has a structure represented by general formula (2):

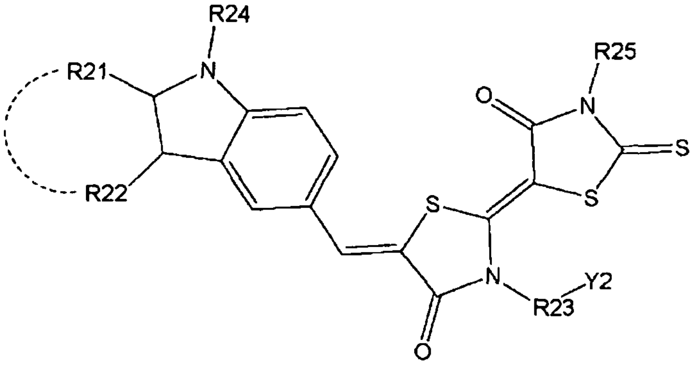

(2)

wherein R21 and R22 each represent a hydrogen atom or an alkyl group, or R21 and R22 are taken together to form a cyclopentane ring or a cyclohexane ring; R23 represents an alkylene group having 1 to 3 carbon atoms; Y2 represents at least one group selected from carboxyl, sulfo, sulfino, sulfeno, phosphono, and phosphinico; R24 represents an aliphatic hydrocarbon residue, an aromatic hydrocarbon residue, or a heterocyclic residue; R25 represents an alkyl group or an aralkyl group, provided that at least one of R24 and R25 contains at least one group selected from carboxyl, sulfo, sulfino, sulfeno, phosphono, and phosphinico bonded via an alkylene group having more than 3 carbon atoms.

**5.** A method for making the dye-sensitized solar cell according to one of claims 1 to 4 comprising the steps of:

providing a substrate (1) having a transparent conductive layer (2),
forming a metal oxide porous layer (5) composed of metal oxide particles (6/7) on the transparent conductive layer (2),
causing an organic cyanine dye (8) to be adsorbed onto the metal oxide porous layer (5), **characterized in that** the method comprises the step of:

causing a dye containing an indoline structure (9) to be adsorbed onto the metal oxide porous layer (5) having the organic cyanine dye (8) adsorbed thereon.

## Patentansprüche

**1.** Farbstoffsensibilisierte Solarzelle, umfassend eine Photoelektrode, welche ein Substrat (1), eine transparente leitfähige Schicht (2) auf dem Substrat (1) und eine poröse Metalloxid- Schicht (5), die Metalloxid- Teilchen (6/7) enthält, auf der transparenten leitfähigen Schicht (2) umfasst, wobei die poröse Metalloxid- Schicht (5) sensibilisierende Farbstoffe aufweist, die daran adsorbiert sind, **dadurch gekennzeichnet, dass**
die sensibilisierenden Farbstoffe einen organischen Cyaninfarbstoff (8) und einen Indolinfarbstoff, der eine Indolinstruktur (9) enthält, umfassen,
der organische Cyaninfarbstoff (8) und der Indolinfarbstoff (9) an die poröse Metalloxid- Schicht (5) in einer Vielzahl von Ebenen adsorbiert sind, und
der organische Cyaninfarbstoff (8) in einer höheren Konzentration als der Indolinfarbstoff (9) auf der Oberfläche der Metalloxid- Teilchen (7) vorhanden ist.

**2.** Die farbstoffsensibilisierte Solarzelle nach Anspruch 1, wobei der organische Cyaninfarbstoff (8) und der Indolinfarbstoff (9) jeweils mindestens eine Gruppe aufweisen, die ausgewählt ist aus einer Carboxyl-, einer Sulfo-, einer Sulfino-, einer Sulfeno-, einer Phosphono- und einer Phosphinico- Gruppe.

**3.** Die farbstoffsensibilisierte Solarzelle nach Anspruch 1 oder 2, wobei der organische Cyaninfarbstoff (8) eine Struktur aufweist, die durch die allgemeinen Formel (1) wiedergegeben ist:

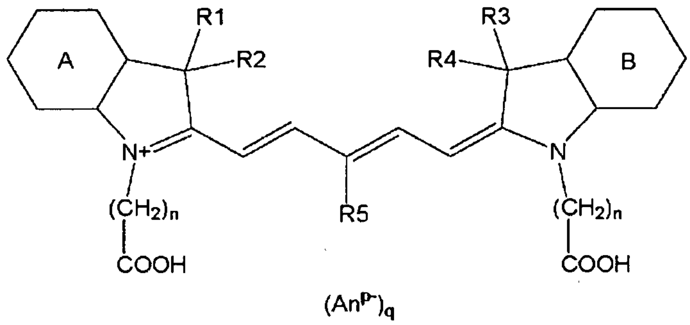

(1)

wobei der Ring A und der Ring B jeweils unabhängig voneinander einen gegebenenfalls substituierten Benzol- oder Naphthalinring darstellen; R1, R2, R3 und R4 jeweils unabhängig voneinander eine Alkylgruppe mit 1 bis 10 Kohlenstoffatomen oder eine gegebenenfalls substituierte Benzylgruppe darstellen; R5 eine Cyanogruppe, ein Fluoratom, ein Chloratom, ein Bromatom oder ein Iodatom darstellt; jedes n jeweils unabhängig voneinander eine ganze Zahl von 1 bis 3 darstellt; $An^{p-}$ ein p-wertiges Anion darstellt, p die ganze Zahl 1 oder 2 darstellt; q eine ganze Zahl von 0 bis 2 darstellt.

4. Die farbstoffsensibilisierte Solarzelle nach Anspruch 1, 2 oder 3, wobei der Indolinfarbstoff (9) eine Struktur aufweist, die durch die allgemeine Formel (2) wiedergegeben ist:

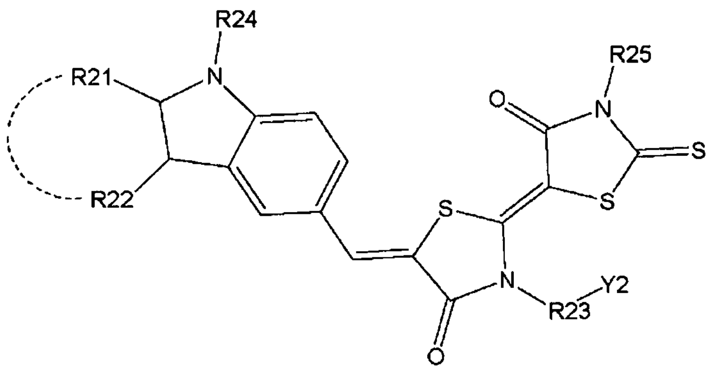

(2)

wobei R21 und R22 jeweils ein Wasserstoffatom oder eine Alkylgruppe darstellen, oder R21 und R22 miteinander verbunden sind, um einen Cyclopentanring oder einen Cyclohexanring zu bilden; R23 eine Alkylengruppe mit 1 bis 3 Kohlenstoffatomen darstellt; Y2 mindestens eine Gruppe darstellt, die ausgewählt ist aus einer Carboxyl-, einer Sulfo-, einer Sulfino-, einer Sulfeno-, einer Phosphono- und einer Phosphinico- Gruppe; R24 einen aliphatischen Kohlenwasserstoffrest, einen aromatischen Kohlenwasserstoffrest oder einen heterocyclischen Rest darstellt; R25 eine Alkylgruppe oder eine Aralkylgruppe darstellt, mit der Maßgabe, dass mindestens einer der Reste R24 und R25 mindestens eine Gruppe enthält, die ausgewählt ist aus einer Carboxyl-, einer Sulfo-, einer Sulfino-, einer Sulfeno-, einer Phosphono- und einer Phosphinico- Gruppe, welche über eine Alkylengruppe mit mehr als 3 Kohlenstoffatomen gebunden ist.

5. Verfahren zur Herstellung der farbstoffsensibilisierten Solarzelle nach einem der Ansprüche 1 bis 4, welches die Schritte umfasst:

das Bereitstellen eines Substrats (1), welches eine transparente leitfähige Schicht (2) aufweist,
das Bilden einer porösen Metalloxid- Schicht (5), die aus Metalloxid- Teilchen (6/7) auf der transparenten leitfähigen Schicht (2) zusammengesetzt ist,
bewirken, dass ein organischer Cyaninfarbstoff (8) an der porösen Metalloxid- Schicht (5) adsorbiert wird, **dadurch gekennzeichnet, dass** das Verfahren den folgenden Schritt umfasst:

bewirken, dass ein Farbstoff, der eine Indolinstruktur (9) enthält, an die poröse Metalloxid- Schicht (5), welche einen daran adsorbierten organischen Cyaninfarbstoff (8) aufweist, adsorbiert wird.

## Revendications

1. Cellule solaire sensibilisée par un colorant, comprenant une photo-électrode comprenant un substrat (1), une couche conductrice transparente (2) sur le substrat (1) et une couche poreuse d'un oxyde métallique (5) contenant des particules d'oxyde métallique (6/7) sur la couche conductrice transparente (2), la couche poreuse d'oxyde métallique (5) présentant des colorants de sensibilisation adsorbés sur celle-ci, **caractérisée en ce que**
les colorants de sensibilisation comprennent un colorant organique cyanine (8) et un colorant indoline contenant une structure indoline (9),
le colorant organique cyanine (8) et le colorant indoline (9) étant adsorbés sur la couche poreuse d'oxyde métallique (5) en une pluralité de degrés, et
le colorant organique cyanine (8) étant présent en une concentration plus élevée que le colorant indoline (9) sur la surface des particules d'oxyde métallique (7).

2. Cellule solaire sensibilisée par un colorant selon la revendication 1, dans laquelle le colorant organique cyanine (8) et le colorant indoline (9) ont chacun au moins un groupe choisi parmi carboxyle, sulfo, sulfino, sulféno, phosphono, et phosphinico.

3. Cellule solaire sensibilisée par un colorant selon la revendication 1 ou 2, dans laquelle le colorant organique cyanine (8) possède une structure représentée par la formule générale (1) :

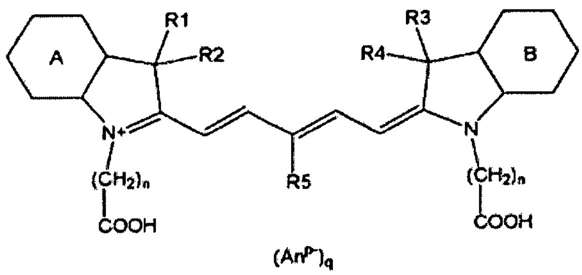

(1)

dans laquelle le cycle A et le cycle B représentent, chacun indépendamment, un cycle benzène ou naphtalène éventuellement substitué ; R1, R2, R3 et R4 représentent, chacun indépendamment, un groupe alkyle ayant 1 à 10 atomes de carbone ou un groupe benzyle éventuellement substitués ; R5 représente un groupe cyano, un atome de fluor, un atome de chlore, un atome de brome, ou un atome d'iode ; les n représentent, chacun indépendamment, un entier égal à un nombre de 1 à 3 ; $An^{p-}$ représente un anion de valence p, p représente un entier égal à 1 ou 2 ; q représente un entier égal à un nombre de 0 à 2.

4. Cellule solaire sensibilisée par un colorant selon la revendication 1, 2 ou 3, dans laquelle le colorant indoline (9) a une structure représentée par la formule générale (2) :

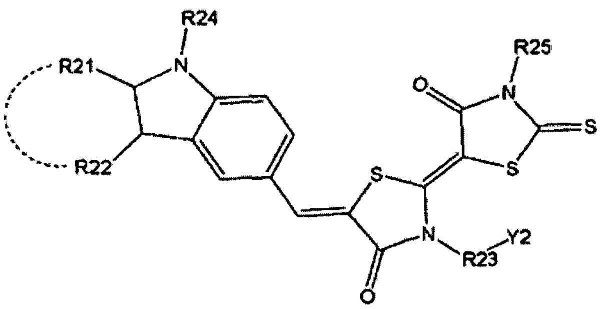

(2)

dans laquelle R21 et R22 représentent chacun un atome d'hydrogène ou un groupe alkyle, ou R21 et R22, pris conjointement, forment un cycle cyclopentane ou un cycle cyclohexane ; R23 représente un groupe alkylène ayant de 1 à 3 atomes de carbone ; Y2 représente au moins un groupe choisi parmi carboxyle, sulfo, sulfino, sulféno, phosphono, et phosphinico ; R24 représente un résidu hydrocarboné aliphatique, un résidu hydrocarboné aromatique ou un résidu hétérocyclique ; R25 représente un groupe alkyle ou aralkyle, à condition qu'au moins un parmi R24 et R25 contienne au moins un groupe choisi parmi carboxyle, sulfo, sulfino, sulféno, phosphono, et phosphinico lié par le biais d'un groupe alkylène présentant plus de 3 atomes de carbone.

**5.** Procédé de fabrication de la cellule solaire sensibilisée par un colorant selon l'une des revendications 1 à 4, comprenant les étapes:

fournir un substrat (1) ayant une couche conductrice transparente (2),
former une couche poreuse d'oxyde métallique (5) constituée de particules d'oxyde métallique (6/7) sur la couche conductrice transparente (2),
provoquer l'adsorption d'un colorant organique cyanine (8) sur la couche poreuse d'oxyde métallique (5), **caractérisé en ce que** le procédé comprend l'étape consistant à :

provoquer l'adsorption d'un colorant contenant une structure indoline (9) sur la couche poreuse d'oxyde métallique (5) présentant le colorant organique cyanine (8) adsorbé sur celle-ci.

[Fig.1]

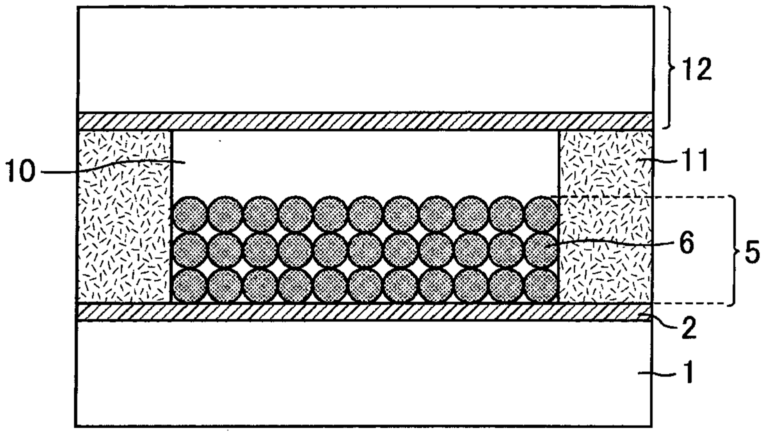


[Fig.2]

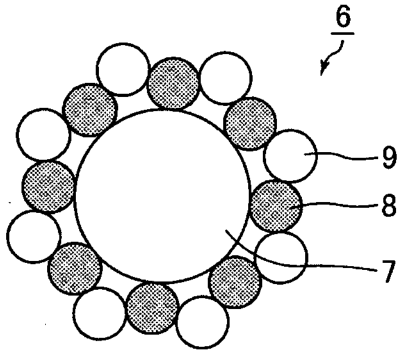


[Fig.3]

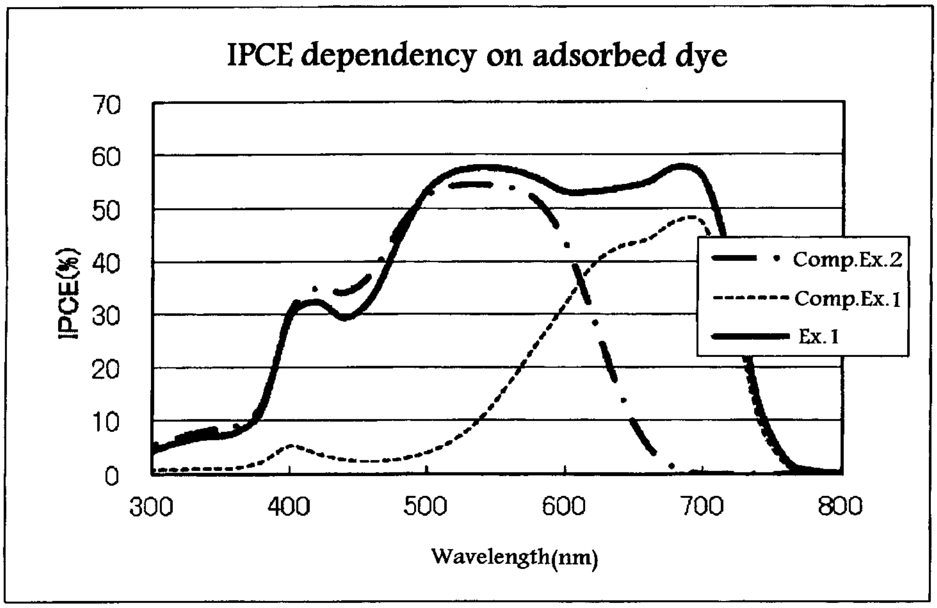

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2664194 B
- JP 3505414 B
- JP 4574897 B
- JP 2009032547 A
- EP 2037528 A1
- US 2012012775 A1
- JP 4692694 B
- JP 9507334 A
- JP 8259543 A
- JP 2005093313 A
- JP 2003331936 A
- JP 2003168493 A


### Non-patent literature cited in the description


- **SAYAMA K et al.** *SOLAR ENERGY MATERIALS AND SOLAR CELLS,* 2003, vol. 80 (1), 47-71
- **GUO M et al.** *SOLAR ENERGY MATERIALS AND SOLAR CELLS,* 2005, vol. 88 (1), 23-35
- *Inorg. Chem.,* 1996, vol. 35, 1168-1178
- *Electrochemistry,* 2002, vol. 2, 130-136